(19) Europäisches Patentamt
European Patent Office
Office européen des brevets

(11) Veröffentlichungsnummer: **0 361 272 B1**

(12) **EUROPÄISCHE PATENTSCHRIFT**

(45) Veröffentlichungstag der Patentschrift: **01.06.94**

(51) Int. Cl.5: **C07D 405/12**, C07D 317/30, C07D 405/14, C09K 19/34, G02F 1/13, G09F 9/35

(21) Anmeldenummer: **89117265.2**

(22) Anmeldetag: **19.09.89**

# (54) Optische aktive Ester der 5-Ethyl- und der 5-Vinyl-1,3-dioxolan-4-carbonsäure, ihre Verwendung als Dotierstoffe in Flüssigkristallmischungen und die Ester enthaltende Flüssigkristallmischungen.

(30) Priorität: **24.09.88 DE 3832503**

(43) Veröffentlichungstag der Anmeldung:
**04.04.90 Patentblatt 90/14**

(45) Bekanntmachung des Hinweises auf die Patenterteilung:
**01.06.94 Patentblatt 94/22**

(84) Benannte Vertragsstaaten:
**AT BE CH DE FR GB IT LI NL SE**

(56) Entgegenhaltungen:
**EP-A- 0 307 880**
**DE-A- 3 604 898**
**DE-A- 3 713 273**

**CHEMICAL ABSTRACTS, Band 110, Nr. 20, 25. Mai 1989, Columbus, Ohio, USA; H.R. DUEBAL et al.: "Three classes of new chiral dopants: synthesis and physical qualification as dopants for practical ferroelectric liquid crystal mixtures", Seite 709, Spalte 2, Zusammenfassung-Nr. 183 512a**

(73) Patentinhaber: **HOECHST AKTIENGESELLSCHAFT**

**D-65926 Frankfurt(DE)**

(72) Erfinder: **Müller, Ingrid, Dr.**
**Am Pfingstbrunnen 1**
**D-6238 Hofheim am Taunus(DE)**
Erfinder: **Dübal, Hans-Rolf, Dr.**
**Heuhohlweg 6**
**D-6240 Königstein/Taunus(DE)**
Erfinder: **Escher, Claus, Dr.**
**Amselweg 3**
**D-6109 Mühltal(DE)**
Erfinder: **Hemmerling, Wolfgang, Dr.**
**Billtalstrasse 32**
**D-6231 Sulzbach(DE)**
Erfinder: **Illian, Gerhard, Dr.**
**Rauenthaler Weg 32**
**D-6000 Frankfurt am Main 71(DE)**
Erfinder: **Murakami, Mikio**
**Bahnstrasse 2c**
**D-6240 Königstein/Taunus(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

Erfinder: **Ohlendorf, Dieter, Dr.**
**Am Kühlen Grund 4**
**D-6237 Liederbach(DE)**
Erfinder: **Wingen, Rainer, Dr.**
**Rotkäppchenweg 10**
**D-6234 Hattersheim am Main(DE)**

## Beschreibung

Gegenstand der europäischen Patentanmeldung 0 288 813 sind optisch aktive 1,3-Dioxolan-4-carbonsäureester. Als Dotierstoffe bewirken sie schon in kleinen Mengen in geneigt smektischen Phasen kurze Schaltzeiten und in orthogonal smektischen Phasen hohe elektrokline Koeffizienten. Insbesondere ist die Ganghöhe der durch die Dotierung induzierten Helix so groß, daß eine Kompensation durch andere Dotierstoffe nicht nötig ist. Es handelt sich dabei um Verbindungen der allgemeinen Formel

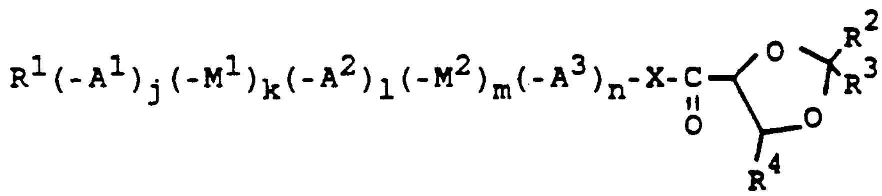

in der die Symbole folgende Bedeutung haben:

$R^1$

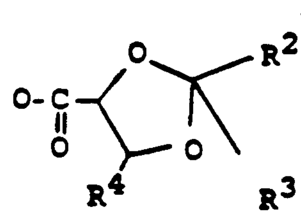

oder

ein geradkettiger oder verzweigter Alkylrest mit 1 bis 16 C-Atomen oder ein geradkettiger oder verzweigter Alkenylrest mit 3 bis 16 C-Atomen, wobei diese Reste selbst asymmetrische C-Atome enthalten können, wobei eine oder mehrere nicht benachbarte $-CH_2$-Gruppen durch -O-, -S-,

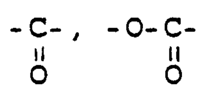 und/oder 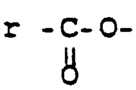

und wobei ein oder mehrere H durch F, Cl, Br oder CN ersetzt sein können

$R^2$, $R^3$ — jeweils H oder ein Alkylrest mit 1 bis 10 C-Atomen, wobei ein oder mehrere H durch F ersetzt sein können, oder $R^2$ und $R^3$ bzw. $R^{2'}$ und $R^{3'}$ bilden zusammen mit dem C(2)-Atom des Dioxolanrings einen Cyclopentan-, Cyclohexan- oder Cycloheptanring

$R^4$, — H oder ein Alkylrest mit 1 bis 10 oder ein Alkenylrest mit 2 bis 10 C-Atomen

j und 1 — null, 1 oder 2

k und m — null oder 1

n — null, 1 oder 2

mit folgender Maßgabe: wenn j und/oder l = null sind, ist k = null; wenn n = null ist, ist m = null; die Summe j + l + n ist minimal 1 und maximal 3,

$-A^1$ und $-A^2$

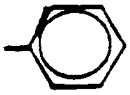, 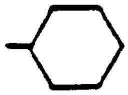, 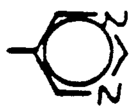, 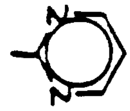,

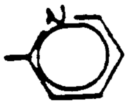, 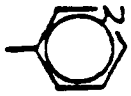, 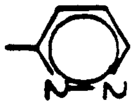, 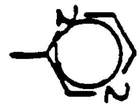,

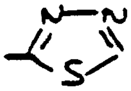, 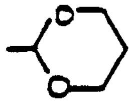, 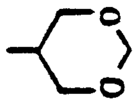, 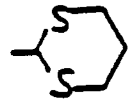,

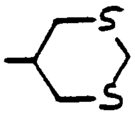, 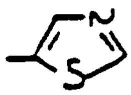, 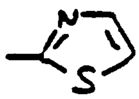, 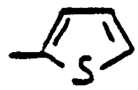,

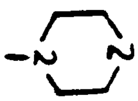

-$A^3$

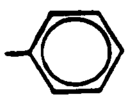, 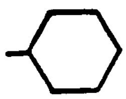, 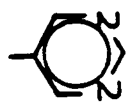, 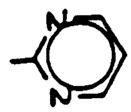,

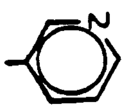, 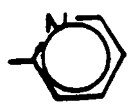, 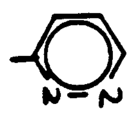

-$M^1$ und -$M^2$

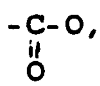, 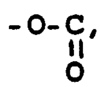,

$-CH_2CH_2$, $-CH=CH$, $-CH_2O$, $-OCH_2$

X O oder S.

In einer bevorzugten Ausführungsform haben die Symbole in der allgemeinen Formel (I) die folgende Bedeutung:

$R^1$ ein geradkettiger oder verzweigter Alkyl- oder Alkenylrest mit 4 bis 14 C-Atomen, der ein asymmetrisches C-Atom enthalten kann, oder wobei eine $-CH_2$-Gruppe durch -O-,

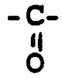 oder 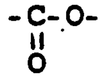

ersetzt sein kann,
oder wobei ein oder mehrere H durch F ersetzt sein können,

$R^2$, $R^3$, $R^4$ H oder ein Alkylrest mit 1 bis 5 C-Atomen oder $R^2$, $R^3$ zusammen mit dem C(2)-Atom des Dioxolanrings einen Cyclopentan- oder Cyclohexanring,

j und l null oder 1,

k, m, n null oder 1

$-M^1$, $-M^2$

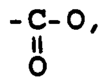, 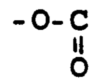

X O oder S.

In einer weiteren bevorzugten Ausführungsform werden 1,3-Dioxolan-4-carbonsäureester der allgemeinen Formel (IV) eingesetzt

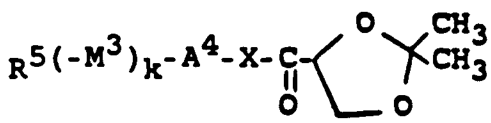 (IV)

worin bedeuten:

$R^5$ ein geradkettiger oder verzweigte Alkyl- oder Alkenylrest mit 6 bis 12 C-Atomen, der ein asymmetrisches C-Atom enthalten kann,

$-M^3$ -O, -S,

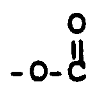 oder 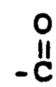

$-A^4$

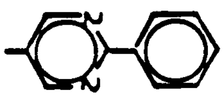 , 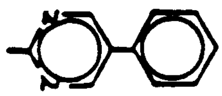 , 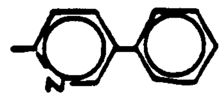 ,

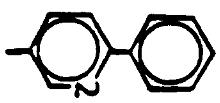 , 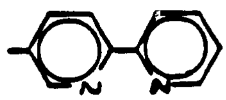 , 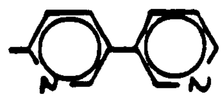 ,

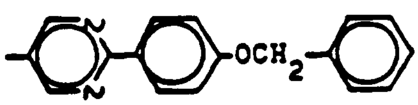 , 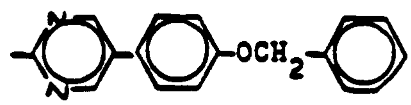 ,

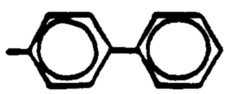 , 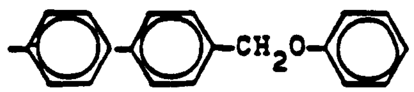 ,

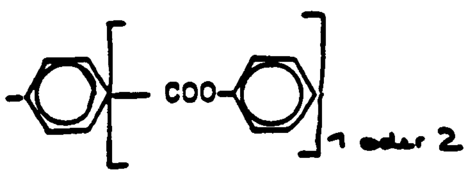

Die Werte für die spontane Polarisation (Ps) liegen für die in der DE-Patentanmeldung beschriebenen Verbindungen bei 25° C und 10 Mol-% Dotierung von geneigt-smektischer Flüssigkristallphase im Bereich von etwa 8 - 14 $nC/cm^2$ und im Bereich von etwa 80 - 140 $nC/cm^2$ linear extrapoliert auf die reine Verbindung.

Es wurde nun überraschend gefunden, daß die Werte für Ps in geneigt-smektischen Flüssigkristallphasen bei gleicher Wirtsubstanz gegenüber der Dotierung mit den vorgenannten Verbindungen um 50 bis 100 % höher liegen, wenn man als Dotierstoffe 1,3-Dioxolan-4-carbonsäureester der allgemeinen Formel (V)

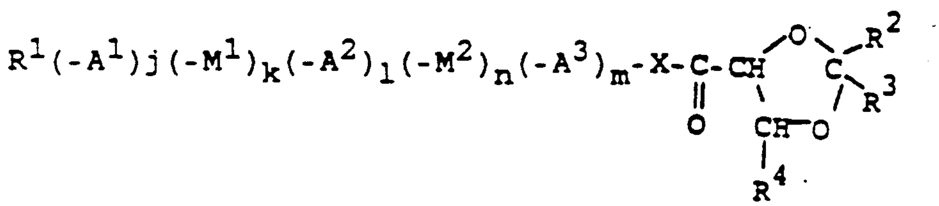 (V)

oder (VI)

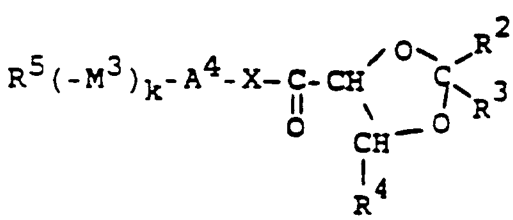 (VI)

verwendet, in denen $R^1$, $R^2$, $R^3$, $R^5$, $A^1$, $A^2$, $A^3$, 4, $M^1$, $M^2$, $M^3$, X, j, k, l, m und n die gleiche Bedeutung haben wie bei Formel (I) und (IV) angegeben und in denen $R^4$ ein Ethyl-ist, wobei in Formel (V), wenn $R^1$ ein Dioxolan-4-carboxylrest ist, einer oder beide Reste $R^4$ ein Ethyl- rest ist und der andere ein Rest $R^4$ mit der bei Formel (I) angegebenen Bedeutung sein kann. $R^2$ und $R^3$ in Formel (V) und (VI) sind bevorzugt beide $CH_3$ oder bilden zusammen mit dem $C_2$-Atom des Dioxolanrings einen Cyclohexanring.

Zu den neuen Verbindungen der Formel (V), insbesondere (VI) gehören bevorzugt die in den Beispielen namentlich genannten Verbindungen. Zur Herstellung der Verbindungen der Formel (V) bzw. (VI) werden mesogene Phenole bzw. Thiophenole der Formel (II)

$$R^1\text{-}(\text{-}A^1)_j(\text{-}M^1)_k(\text{-}A^2)_l(\text{-}M^2)_m(\text{-}A^3)_nXH \qquad \text{(II)}$$

bzw. der Formel (IIa)

$$R^5(\text{-}M^3)_k\text{-}A^4\text{-}XH \qquad \text{(IIa)}$$

mit Derivaten der 5-Ethyl-1,3-dioxolan-4-carbonsäure (III)

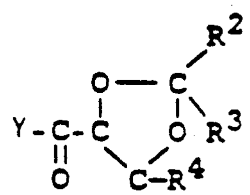

(III)

in der $R^4$ ein Ethyl- rest ist, in Gegenwart von Kondensationsmitteln umgesetzt (vgl. z. B. March, Advanced Organic Chemistry, 2nd Edition, Mc Graw-Hill, S. 363 - 365), und das Reaktionsprodukt wird isoliert und z. B. durch Umkristallisieren oder chromatographische Trennverfahren gereinigt.

Die zu verwendenden Phenole sind literaturbekannt wie auch Verfahren zum Herstellen der 5-Vinyl-1,3-dioxolan-4-carbonsäure und ihrer Derivate (vgl. Synthesis 1987 (9), 801 - 806). Die entsprechenden 5-Ethyl-Verbindungen erhält man nach bekannten Methoden, z. B. durch Hydrieren in Gegenwart eines Pd-Kohle-Katalysators.

Die Flüssigkristallmischungen gemäß der Erfindung bilden Flüssigkristall-Phasen und enthalten mindestens eine optisch aktive Verbindung der allgemeinen Formel (V) bzw. (VI).

Unter dem Begriff "Flüssigkristallphase" sind nematische, cholesterische, orthogonal smektische oder geneigt ("tilted")-smektische, insbesondere $S_A$-, $S_B$- und $S_C$-Phasen zu verstehen. Die Flüssigkristallmischungen bestehen aus 2 bis 20, vorzugsweise 2 bis 15 Komponenten, darunter mindestens eine der erfindungsgemäß beanspruchten chiralen Verbindungen.

Die anderen Bestandteile werden vorzugsweise ausgewählt aus den bekannten Verbindungen mit nematischen, cholesterischen und/oder smektischen, z. B. $S_A$-Phasen, und/oder geneigt smektischen Phasen; dazu gehören beispielsweise Schiff'sche Basen, Biphenyle, Terphenyle, Phenylcyclohexane, Pyrimidine, Zimtsäureester, Cholesterinester, verschiedene überbrückte, terminalpolar mehrkernige Ester von p-Alkylbenzoesäuren. Im allgemeinen liegen die im Handel erhältlichen Flüssigkristallmischungen bereits vor der Zugabe der optisch aktiven Verbindung(en) als Gemische verschiedenster Komponenten vor, von denen mindestens eine mesogen ist, d. h. als Verbindung, in derivatisierter Form oder im Gemisch mit bestimmten Cokomponenten eine Flüssigkristallphase zeigt, die mindestens eine enantiotrope (Klärtemperatur > Schmelztemperatur) oder monotrope (Klärtemperatur < Schmelztemperatur) Mesophasenbildung erwarten läßt. Insbesondere enthält die Flüssigkristall-Mischung neben mindestens einer der erfindungsgemäß beanspruchten optisch aktiven Verbindungen eine Esterverbindung mit $S_C$-Phase, z. B. einen Alkoxybenzoesäurephenylester, oder eine biaromatische Verbindung mit einem stickstoffhaltigen Heterocyclus, z. B. ein Alkylpyrimidinyl-alkoxy-benzol.

Vor der oder den erfindungsgemäßen Verbindung(en) enthalten die Flüssigkristallmischungen im allgemeinen 0,05 bis 70 Gew.-%, insbesondere 0,1 bis 50 Gew.-%. Die erfindungsgemäßen Verbindungen sind insbesondere als Dotierstoffe für geneigt-smektische Flüssigkristallphasen geeignet, da sie diese ein ferroelektrische Flüssigkristallphasen umwandeln; die Werte für die spontane Polarisation (Ps) bei 25° C liegen bei 10 Mol-% Dotierung im Bereich von etwa 29 - 38 $nC/cm^2$ und im Bereich von etwa 290 - 380 $nC/cm^2$ linear extrapoliert auf die reine Verbindung.

Die Schaltzeiten der neuen Systeme liegen meistens deutlich unter 50 $\mu s$ bei 10 Mol-% Dotierung, 25° C

und einer Schaltspannung von ± 10 V/μm.
Die erfindungsgemäßen Verbindungen können auch zur Erzielung des elektroklinen Effektes in orthogonalen smektischen Phasen ($S_A$, $S_B$, $S_E$) eingesetzt werden.

Beispiel 1

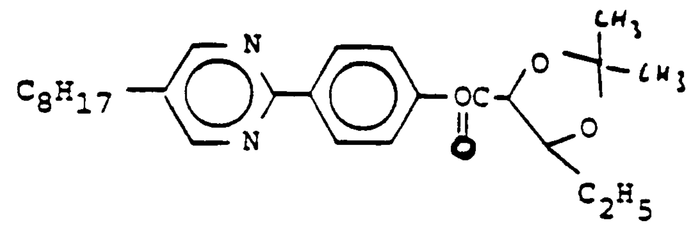

(4R, 5R)-2,2-Dimethyl-5-ethyl-1,3-dioxolan-4-carbonsäure-[4-(5-octyl-pyrimidin-2-yl)-phenyl]-ester.
Schmelzpunkt: 53 ° C

$$[\alpha]_{365}^{22} = -68.8° \ (c = 2, CH_2Cl_2)$$

Beispiel 2

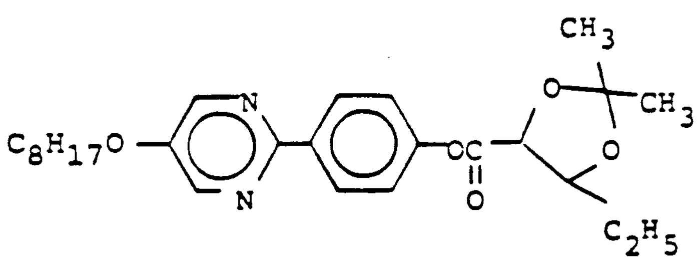

(4R, 5R)-2,2-Dimethyl-5-ethyl-1,3-dioxolan-4-carbonsäure-[4-(5-octyloxy-pyrimidin-2-yl)-phenyl]-ester.
Schmelzpunkt: 87 ° C

$$[\alpha]_{365}^{22} = -70.5° \ (c = 2, CH_2Cl_2)$$

Beispiel 3

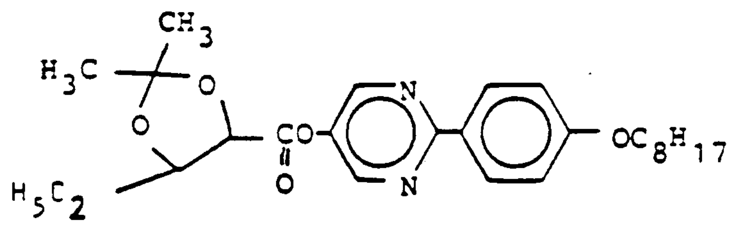

(4R, 5R)-2,2-Dimethyl-5-ethyl-1,3-dioxolan-4-carbonsäure-[2-(4-octyloxy-phenyl)-pyrimidin-5-yl]-ester. Schmelzpunkt: 128° C

$$[\alpha]_{365}^{22} = -63.4° \ (c = 2, CH_2Cl_2)$$

Beispiel 4

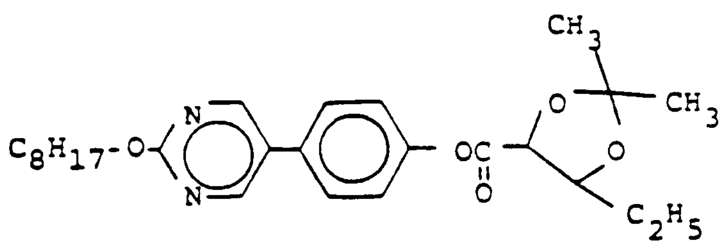

(4R, 5R)-2,2-Dimethyl-5-ethyl-1,3-dioxolan-4-carbonsäure-[4-(2-octyloxy-pyrimidin-5-yl)-phenyl]-ester. Schmelzpunkt: 110° C

$$[\alpha]_{365}^{22} = -64.3° \ (c = 2, CH_2Cl_2)$$

Meßmethode:

Versetzt man ein (nicht-chirales) Lösemittel mit einer kleinen Menge einer chiralen Verbindung, so wird die Ebene des linear polarisierten Lichts um den (charakteristischen) Winkel $\alpha$ gedreht; dieser Winkel wird wie folgt angegeben:
$[\alpha]_\lambda^T$ (c = x, LM), wobei die Symbole folgende Bedeutung haben: $\lambda$: Wellenlänge [nm] des polarisierten Lichtes, x = Konzentration der Lösung in g/l, LM = Lösemittel, T = Temperatur der Lösung. Der Drehwinkel wird in einem Polarimeter nach 10 cm Durchgang des Lichts bestimmt.

**Anwendungsbeispiele A1 bis A4**

Zur Überprüfung der Wirksamkeit der vorstehend beschriebenen Verbindung als ferroelektrische Dotierstoffe in Flüssigkristall-Systemen mit geneigten smektischen Phasen werden diese in Konzentrationen von jeweils 10 Mol-% mit einer nicht-chiralen Testmischung mit der Phasenfolge K 12,5° C $S_c$ 83°C $S_A$ 95° N 100° C I gemischt und die Werte für die spontante Polarisation ($P_s$ in nC/cm$^2$) der Mischung bestimmt. Die $P_s$-Werte werden nach der Methode von H. Diamant et al. (Rev. Sci. Instr., 28, 30, 1957) gemessen, wobei

eine spezielle Meßzelle [Skarp et al. in Ferroelectric Letters Vol. 06, 67 (1986)] verwendet wird. Bei einer Zellenschichtdicke von ca. 2 µm wird durch Scherung eine einheitliche planare Orientierung der Flüssigkristalle in der $S_c$-Phase erreicht [SSFLC-Technik, Clark et al., Appl. Phys. Lett. 36, 899 (1980)]. In Tabelle 1 ist neben den Werten für $P_s$ der $S_c$-Bereich der jeweiligen Mischung angegeben. In Tabelle 2 sind in der zweiten Spalte Verbindungen gemäß der deutschen Patentanmeldung P 37 13 273.3 erfindungsgemäßen Verbindungen (dritte und vierte Spalte) gegenübergestellt, wobei die $P_s$ in der gleichen Wirtsubstanz bei gleicher Dotierungshöhe (10 Mol-%) und gleicher Temperatur (25° C) verglichen wird. Diese Beispiele zeigen, daß unter sonst gleichen Bedingungen der Wert der spontanen Polarisation gegenüber der in 5-Stellung des Dioxolanrings unsubstituierten Verbindung bei der 5-Ethyl- verbindungen um 50 - 100 % höher liegt.

Tabelle 1

| Substanz-beispiel | Anwendungs-beispiel | Phasenbereich der Mischung [° C] K | $S_c$* | $S_A$* | N | I | $P_s$ [nC/cm$^2$] |
|---|---|---|---|---|---|---|---|
| 1 | A1 | · 9,5 | · 68 | · 83,5 | · 95,5 | · | 36 |
| 2 | A2 | · 12 | · 71 | · 89,5 | · 97 | · | 29 |
| 3 | A3 | · 11 | · 71 | · 88 | · 93,5 | · | 33 |
| 4 | A4 | · 11,5 | · 77 | | · 93,5 | · | 31 |

Anwendungsbeispiel A1 ist außerdem dadurch gekennzeichnet, daß der Pitch in der nematischen Phase mit 7,8 µm bei 83,6° C so groß ist, daß eine weiter Kompensation mit einem zweiten Dotierstoff nicht notwendig ist.

**Tabelle 2**

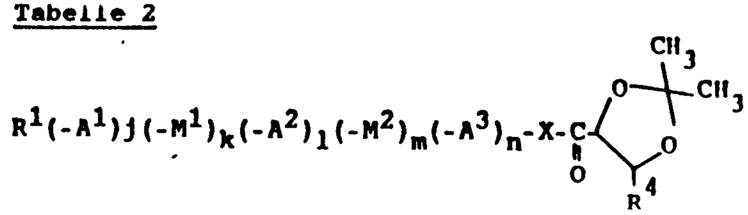

| $R^1(-A^1)_j(-M^1)_k(-A^2)_l(-M^2)_m(-A^3)_n-X$ | | $P_s$ [nC/cm²] 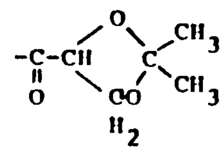 | $P_s$ [nC/cm²] 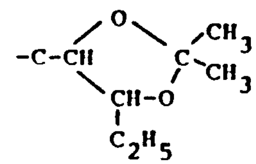 | |
|---|---|---|---|---|
| 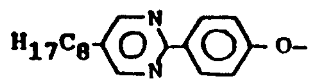 | | 24 | 36 | |
| 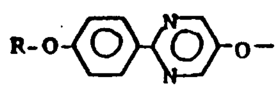 | $R = C_{12}H_{25}$ | 13 | 33 | $R = C_8H_{17}$ |
| 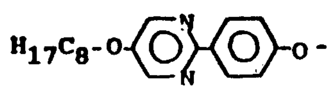 | | 19 | 29 | |
| 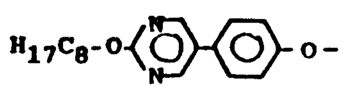 | | 14 | 31 | |

**Anwendungsbeispiel A5**

Eine ferroelektrische Mischung aus

| | |
|---|---|
| 5-Octyl-2-(4-hexyloxyphenyl)-pyrimidin | 14,2 Mol-% |
| 5-Octyl-2-(4-octyloxyphenyl)-pyrimidin | 12,5 Mol-% |
| 5-Octyl-2-(4-decyloxyphenyl)-pyrimidin | 9,4 Mol-% |
| 5-Octyloxy-2-(4-butyloxyphenyl)-pyrimidin | 9,4 Mol-% |
| 5-Octyloxy-2-(4-hexyloxyphenyl)-pyrimidin | 8,9 Mol-% |
| 5-Octyloxy-2-(4-octyloxyphenyl)-pyrimidin | 4,1 Mol-% |
| 5-Octyloxy-2-(4-decyloxyphenyl)-pyrimdin | 7,5 Mol-% |
| trans-4-Pentyl-cyclohexancarbonsäure-[4-(5-dodecyl-pyrimidin--2-yl)]-phenylester | 14 Mol-% |
| (2R,3R)-3-Propyl-oxiran-2-carbonsäure-[4-(2-octyloxypyrimidin--5-yl)-phenyl]-ester | 5,4 Mol-% |
| (2S,3S)-2-[4-(5-Octyl-pyrimidin-2-yl)-phenyloxy]-methyl-3-butyl--oxiran | 13,1 Mol-% |
| (4R,5R)-4-(5-n-Octyl-pyrimidin-2-yl)-phenyl-2,2-dimethyl-5-ethyl--1,3-dioxolan-4-carbonsäureester (Erfindungsgemäßer Dotierstoff) | 1,5 Mol-% |

weist folgende Phasen auf:

X -4 $S_c^*$ 64 $S_A^*$ 69 N*80 I

Bei 20 °C weist diese Mischung eine Polarisation von 49 nC•cm$^{-2}$ auf.

**Anwendungsbeispiel A6**

a) Eine ferroelektrische Mischung aus den 7 Komponenten

| | |
|---|---|
| 5-Octyloxy-2-(4-hexyloxy-phenyl)-pyrimidin | 23 Mol-% |
| 5-Octyloxy-2-(4-octyloxy-phenyl)-pyrimidin | 10,6 Mol-% |
| 5-Octyloxy-2-(4-butyloxy-phenyl)-pyrimidin | 24,3 Mol-% |
| 5-Octyloxy-2-(4-decyloxy-phenyl)-pyrimidin | 19,3 Mol-% |
| trans-4-Pentyl-cyclohexancarbonsäure-[4-(5-decyl-pyrimidin-2-yl)]-phenylester | 13,8 Mol-% |
| (2R,3R)-3-Propyl-oxiran-2-carbonsäure-4-(2-octyloxy-pyrimidin-5-yl)-phenylester | 2 Mol-% |
| Verbindung Beispiel 5 | 7 Mol-% |

weist folgende Phasenfolge auf:

X 9 $S_c$ 71 $S_A$ 85 N 96 I

Die spontane Polarisation bei 20 °C beträgt 43 nC•cm$^{-2}$ und bei einem Feld von 10 V•µm$^{-1}$ beträgt die Schaltzeit ($\tau_{0,90}$) 74 µs.

Anwendungsbeispiel A7

a) Eine ferroelektrische Mischung aus den 8 Komponenten

| | |
|---|---|
| 5-Octyloxy-2-(4-hexyloxy-phenyl)-pyrimidin | 24,8 Mol-% |
| 5-Octyloxy-2-(4-octyloxy-phenyl)-pyrimidin | 11,5 Mol-% |
| 5-Octyloxy-2-(4-butyloxy-phenyl)-pyrimidin | 26,2 Mol-% |
| 5-Octyloxy-2-(4-decyloxy-phenyl)-pyrimidin | 20,8 Mol-% |
| trans-4-Pentyl-cyclohexyncarbonsäure-[4-(5-decylpyrimidin-2-yl)]-phenylester | 14,7 Mol-% |
| (2S,3S)-(-)-2-[4-(5-Octyl-pyrimidin-2-yl)-phenyloxy]-methyl-3-butyloxiran | 1,5 Mol-% |
| (2R,3R)-3-Pentyl-oxiran-2-carbonsäure-[4-(2-octyloxypyrimdin-5-yl)-phenyl]-ester | 0,46 Mol-% |
| Verbindung Beispiel 5 | 0,04 Mol-% |

zeigt folgende flüssigkristalline Phasenbereiche:

X 9 $S_c^*$ 80,4 $S_A^*$ 90 N*102,5 I

und weist bei 25 °C eine spontane Polarisation von 5,6 nC•cm$^{-2}$ und eine Schaltzeit von ($\tau_{0,90}$) von 190 µs auf. Bei einer Temperatur von 95 °C ist der Pitch dieser Mischung > 50 µm.

Die Bestimmung der Schaltzeit $\tau_{0,90}$ erfolgt mit Hilfe einer Photodiode, indem die Anstiegszeit des Lichtsignals von 10 auf 90 % Signalhöhe gemessen wird. Die Schaltspannung besteht aus Rechteckpulsen und beträgt ± 10 V•µm$^{-1}$.

**Anwendungsbeispiel A8**

Eine ferroelektrische Mischung aus

| | |
|---|---|
| 5-Octyl-2-(4-hexyloxyphenyl)-pyrimidin | 13,1 Mol-% |
| 5-Octyl-2-(4-octyloxphenyl)-pyrimidin | 11,6 Mol-% |
| 5-Octyl-2-(4-decyloxphenyl)-pyrimidin | 8,7 Mol-% |
| 5-Octyloxy-2-(4-butyloxphenyl)-pyrimidin | 8,7 Mol-% |
| 5-Octyloxy-2-(4-hexyloxphenyl)-pyrimidin | 8,2 Mol-% |
| 5-Octyloxy-2-[4-octyloxyphenyl)-pyrimidin | 3,8 Mol-% |
| 5-Octyloxy-2-[4-decyloxyphenyl)-pyrimidin | 7 Mol-% |
| trans-4-Pentyl-cyclohexancarbonsäure-[4-(5-dodecyl-pyrimidin-2-yl)]--phenylester | 13 Mol-% |
| trans-4-Pentyl-cyclohexancarbonsäure-[4-(5-decyl-pyrimidin-2-yl)]-phenylester | 3,9 Mol-% |
| (S)-4-(2-Octyloxy-pyrimidin-5-yl)-phenyl-[spiro-(1,3-dioxolan-2,1'-cyclohexan)-4-yl]-methyl-ether | 10 Mol-% |
| (25,3S)-2-[4-(5-Octyl-pyrimidin-2-yl)-phenyloxy]-methyl-3-butyl-oxiran | 10 Mol-% |
| (4R,5R)-4-(5-n-Octyl-pyrimidin-2-yl)-phenyl-2,2-dimethyl-5-ethyl-1,3-dioxolan-4-carbonsäureester (Erfindungsgemäßer Dotierstoff) | 2 Mol-% |

weist folgende Phasen auf:

X -2 $S_c^*$ 60 $S_A^*$ 70 N*78 I

Bei 25 °C weist diese Mischung eine Polarisation von 50 nC•cm$^{-2}$ und bei einem Feld von 10 V•µm$^{-1}$ eine Schaltzeit ($\tau_{0,90}$) von 44 µs auf.

**Patentansprüche**

1. Verwendung von optisch aktiven 1,3-Dioxolan-5--ethyl-4-carbonsäureestern als Dotierstoffe in Flüssigkristallmischungen.

2. Verwendung nach Anspruch 1, dadurch gekennzeichnet, daß 1,3-Dioxolan-4-carbonsäureester der allgemeinen Formel (V) eingesetzt werden,

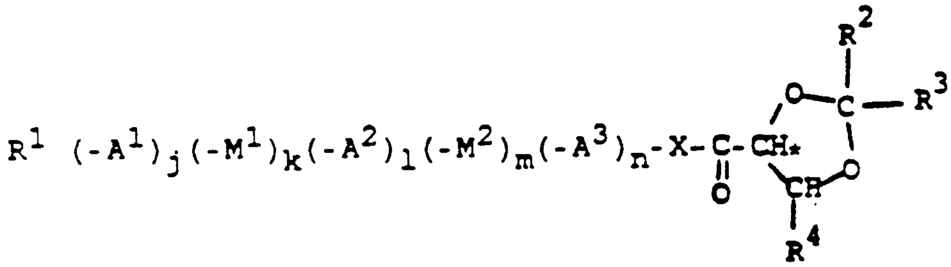

(V),

in der die Symbole folgende Bedeutung haben

$R^1$

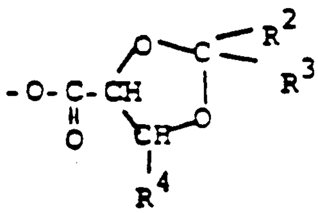

(V a)

oder ein geradkettiger oder verzweigter Alkylrest mit 1 bis 16 C-Atomen oder ein geradkettiger oder verzweigter Alkenylrest mit 3 bis 16 C-Atomen, wobei diese Reste selbst asymmetrische C-Atome enthalten können, wobei eine oder mehrere nicht benachbarte $-CH_2$-Gruppen durch -O-, -S-,

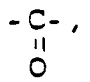 , 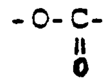 und/oder 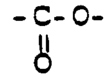

und wobei ein oder mehrere H durch F, Cl, Br oder CN ersetzt sein können

| | |
|---|---|
| $R^2$, $R^3$ | jeweils H oder ein Alkylrest mit 1 bis 10 C-Atomen, wobei ein oder mehrere H durch F ersetzt sein können, oder $R^2$ und $R^3$ bilden zusammen mit dem C(2)-Atom des Dioxolanrings einen Cyclopentan-, Cyclohexan- oder Cycloheptanring |
| $R^4$, | Ethyl oder, falls $R^1$ ein Rest der Formel (Va) ist, H, ein Alkylrest mit 1-10 oder ein Alkenylrest mit 2-10 C-Atomen mit der Maßgabe daß mindestens ein Rest $R^4$ Ethyl ist, |
| j und l | null, 1 oder 2 |
| k und m | null oder 1 |
| n | null, 1 oder 2 |

mit folgender Maßgabe: wenn j und/oder l = null sind, ist k = null; wenn n = null ist, ist m = null; die Summe j + l + n ist minimal 1 und maximal 3, $-A^1$ und $-A^2$

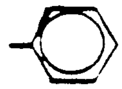 , 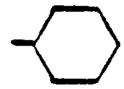 , 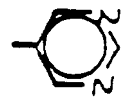 , 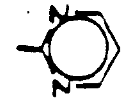 ,

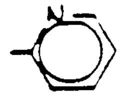 , 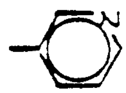 , 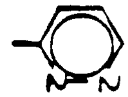 , 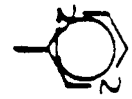 ,

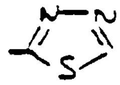 , 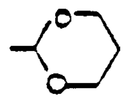 , 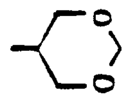 , 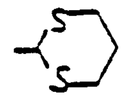 ,

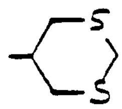 , 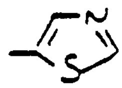 , 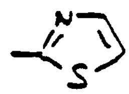 , 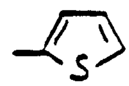 ,

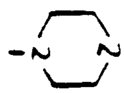

$-A^3$

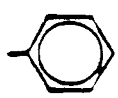 , 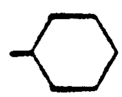 , 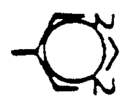 , 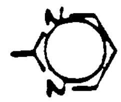 ,

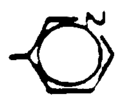 , 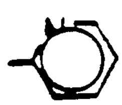 , 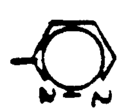

$-M^1$ und $-M^2$

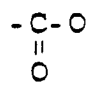 , 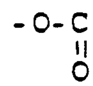 ,

$-CH_2CH_2$, $-CH=CH$, $-CH_2O$, $-OCH_2$

X O oder S.

**3.** Verwendung nach Anspruch 2, dadurch gekennzeichnet, daß 1,3-Dioxolan-4-carbonsäureester der allgemeinen Formel (VI) eingesetzt werden

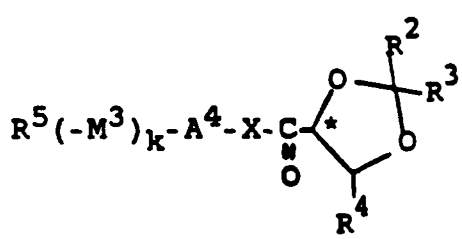 (VI)

worin bedeuten:

$R^4$ Ethyl,

$R^5$ ein geradkettiger oder verzweigter Alkyl- oder Alkenylrest mit 6 bis 12 C-Atomen, der ein

asymmetrisches C-Atom enthalten kann,

-$M^3$ -O, -S,

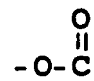 oder 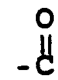

-$A^4$

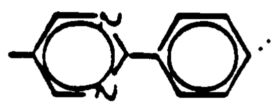 , 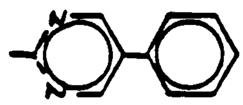 , 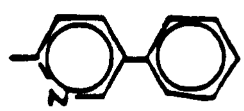 ,

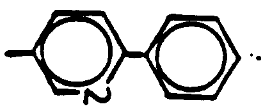 , 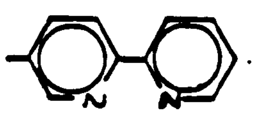 , 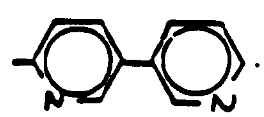 ,

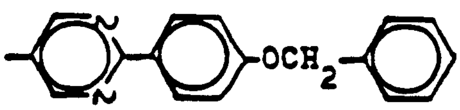 , 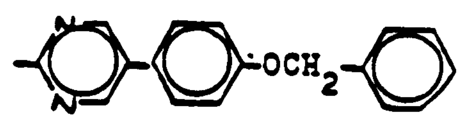 ,

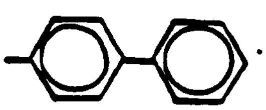 , 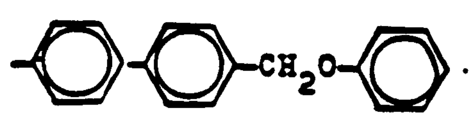 ,

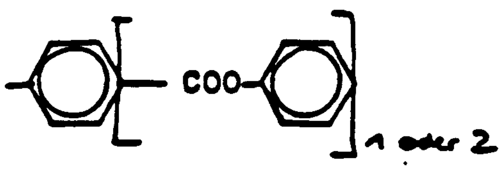

**4.** Verwendung nach Anspruch 2 oder 3, dadurch gekennzeichnet, daß $R^2$ und $R^3$ jeweils $-CH_3$ sind.

**5.** Optisch aktive 1,3-Dioxolan-4-carbonsäureester der allgemeinen Formel (V).

**6.** Optisch aktive 1,3-Dioxolan-4-carbonsäureester der allgemeinen Formel (VI).

**7.** Flüssigkristallmischung, gekennzeichnet durch einen Gehalt an mindestens einem optisch aktiven 1,3-Dioxolan-5-ethyl-4-carbonsäureester.

**8.** Flüssigkristallmischung, gekennzeichnet durch einen Gehalt an mindestens einem optisch aktiven 1,3-Dioxolan-4-carbonsäureester der allgemeinen Formel (V).

**9.** Flüssigkristallmischung, gekennzeichnet durch einen Gehalt an mindestens einem optisch aktiven 1,3-Dioxolan-4-carbonsäureester der allgemeinen Formel (VI).

**10.** Elektrooptisches Schalt- oder Anzeigeelement enthaltend eine Flüssigkristallmischung nach Anspruch 7, 8 oder 9.

**11.** Verfahren zur Herstellung eines optisch aktiven 1,3-Dioxolan-4-carbonsäureesters der allgemeinen Formel (V), dadurch gekennzeichnet, daß mesogene Phenole oder Thiophenole der allgemeinen Formel (II)

$R^1(-A^1)_j(-M^1)_k(-A^2)_l(-M^2)_m(-A^3)_n-X-H$

mit Derivaten der 1,3-Dioxolan-4-carbonsäure (III)

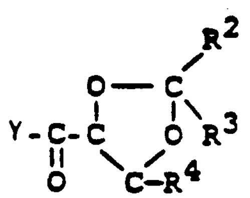

(III)

in der $R^4$ ein Ethyl rest ist, in Gegenwart von Kondensationsmitteln umgesetzt werden, wobei Y eine Abgangsgruppe ist.

## Claims

**1.** Use of optically active 5-ethyl-1,3-dioxolane-4-carboxylic esters as doping substances in liquid crystal mixtures

**2.** Use as claimed in claim 1, wherein 1,3-dioxolane-4-carboxylic esters of the formula (V) are used

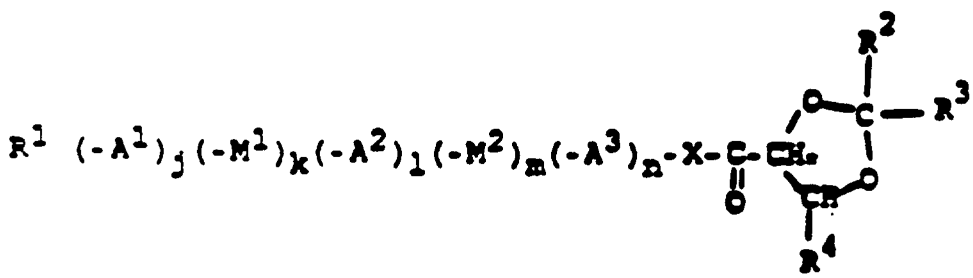

(V)

in which the symbols have the following meaning:

$R^1$ is

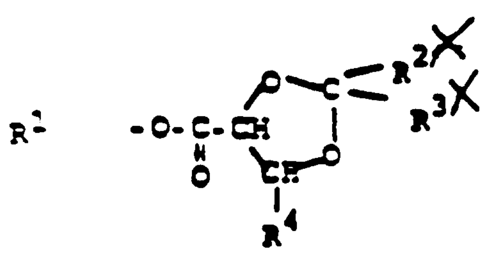

(Va)

or a straight-chain or branched alkyl radical having 1 to 16 carbon atoms or a straight-chain or branched alkenyl radical having 3 to 16 carbon atoms, it being possible for these radicals themselves to contain asymmetric carbon atoms, in which one or more non-adjacent $-CH_2-$ groups can be replaced by -O-, -S-,

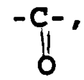, 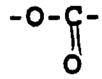 and/or 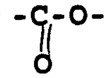

and one or more H can be replaced by F, Cl, Br or CN,

$R^2$ and $R^3$ are each H or an alkyl radical having 1 to 10 carbon atoms, in which one or more H can be replaced by F, or $R^2$ and $R^3$ together with the C(2) atom of the dioxolane ring form a cyclopentane, cyclohexane or cycloheptane ring,

$R^4$ is ethyl or, if $R^1$ is a radical of the formula (Va), $R^4$ is H, an alkyl radical having 1-10 or an alkenyl radical having 2-10 carbon atoms, with the proviso that at least one radical $R^4$ is ethyl,

j and l are zero, 1 or 2,

k and m are zero or 1,

n is zero, 1 or 2,

with the following proviso: if j and/or l are zero, k is zero; if n is zero, m is zero; the sum j + l + n is at least 1 and at most 3, $-A^1$ and $-A^2$ are

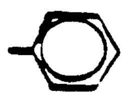, 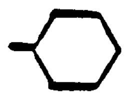, 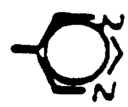, 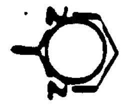,

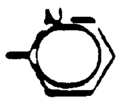, 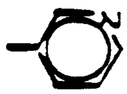, 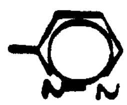, 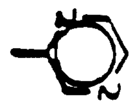,

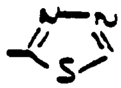, 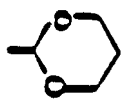, 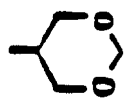, 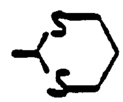,

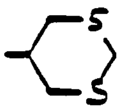, 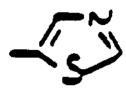, 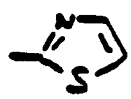, 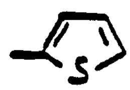,

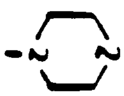

$-A^3$ is

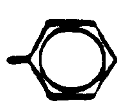, 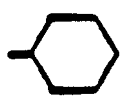, 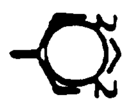, 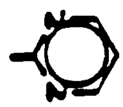,

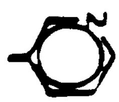, 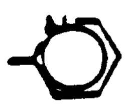, 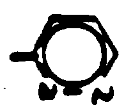

$-M^1$ and $M^2$ are

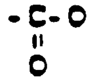, 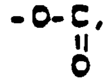

$-CH_2CH_2$, $-CH=CH$, $-CH_2O$, $-OCH_2$ and

X is O or S.

**3.** Use as claimed in claim 2, wherein 1,3-dioxolane-4-carboxylic esters of the formula (VI) are used

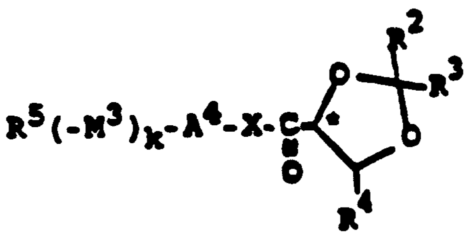 (VI)

in which the symbols have the following meaning:

- $R^4$ is ethyl,
- $R^5$ is a straight-chain or branched alkyl or alkenyl radical having 6 to 12 carbon atoms, which can contain an asymmetric carbon atom,
- $-M^3$ is -O, -S,

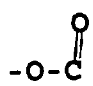 or 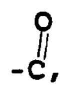,

- $-A^4$ is

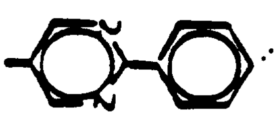, 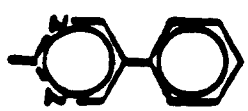, 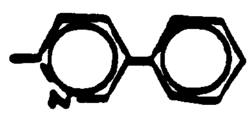,

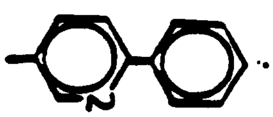; 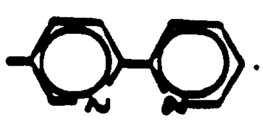, 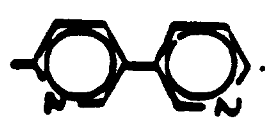,

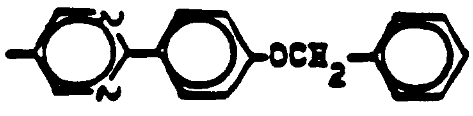,
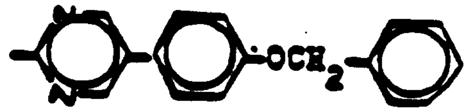,

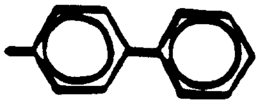,
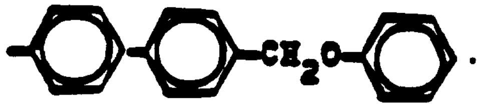,

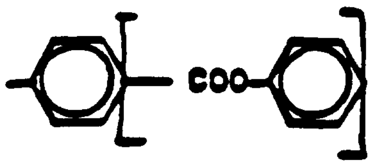

1 or 2

4. Use as claimed in claim 2 or 3, wherein $R^2$ and $R^3$ are each $-CH_3$.

5. An optically active 1,3-dioxolane-4-carboxylic ester of the formula (V).

6. An optically active 1,3-dioxolane-4-carboxylic ester of the formula (VI).

7. A liquid crystal mixture comprising at least one optically active 5-ethyl-1,3-dioxolane-4-carboxylic ester.

8. A liquid crystal mixture comprising at least one optically active 1,3-dioxolane-4-carboxylic ester of the formula (V).

9. A liquid crystal mixture comprising at least one optically active 1,3-dioxolane-4-carboxylic ester of the formula (VI).

10. An electrooptical switching or display element containing a liquid crystal mixture as claimed in claim 7, 8 or 9.

11. A process for the preparation of an optically active 1,3-dioxolane-4-carboxylic ester of the formula (V), which comprises reacting mesogenic phenols or thiophenols of the formula (II)

$R^1(-A^1)_j(-M^1)_k(-A^2)_l(-M^2)_m(-A^3)_n-X-H$

with derivatives of a 1,3-dioxolane-4-carboxylic acid (III)

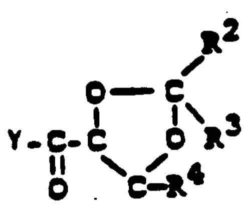

(III),

in which $R^4$ is an ethyl radical, in the presence of condensing agents, Y being a leaving group.

## Revendications

1. Utilisation d'esters de l'acide 1,3-dioxolanne-5-éthyl-4-carboxylique optiquement actifs en tant qu'agents de dopage dans des mélanges de cristaux liquides.

2. Utilisation selon la revendication 1, caractérisée en ce qu'on utilise des esters de l'acide 1,3-dioxolanne-4-carboxylique de formule générale (V) :

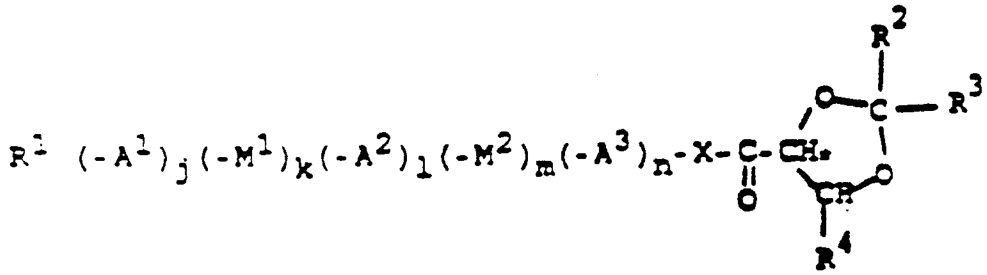

(V),

dans laquelle les symboles ont les significations suivantes :

$R^1$

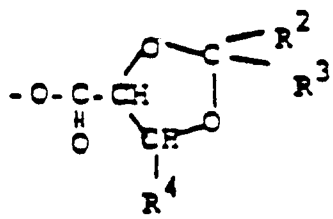

(V a)

ou

un radical alkyle linéaire ou ramifié comportant de 1 à 16 atomes de carbone ou un radical alcényle linéaire ou ramifié comportant de 3 à 16 atomes de carbone, ces radicaux eux-mêmes pouvant contenir des atomes de carbone asymétrique, un ou plusieurs groupes $-CH_2-$ non-voisins pouvant être remplacés par -O-, -S-,

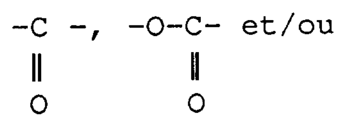

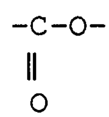

et un ou plusieurs H par F, Cl, Br ou CN,

$R^2$, $R^3$ représentent chaque fois H ou un radical alkyle comportant de 1 à 10 atomes de carbone, un ou plusieurs H pouvant être remplacés par F, ou $R^2$ et $R^3$ forment ensemble avec l'atome C(2) du cycle de dioxolanne un cycle de cyclopentane, de cyclohexane ou de cycloheptane,

$R^4$ est l'éthyle ou, dans le cas où $R^1$ est un radical de formule (Va), H, un radical alkyle comportant de 1 à 10 atomes de carbone ou un radical alcényle comportant de 2 à 10 atomes de carbone, à la condition qu'au moins un radical $R^4$ soit un radical éthyle,

j et l sont zéro, 1 ou 2,

k et m sont zéro ou 1,

n est zéro, 1 ou 2

aux conditions suivantes : lorsque j et/ou l = zéro, k = zéro ; lorsque n = zéro, m = zéro ; la somme j + l + n est au minimum 1 et au maximum 3,

$-A^1$ et $-A^2$

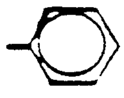 , 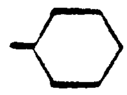 , 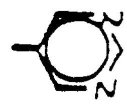 , 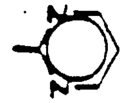 ,

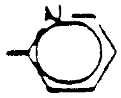 , 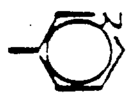 , 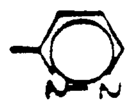 , 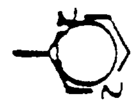 ,

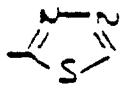 , 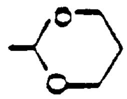 , 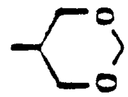 , 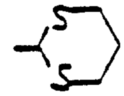 ,

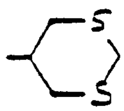 , 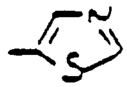 , 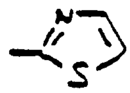 , 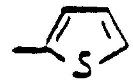 ,

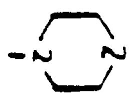

$-A^3$

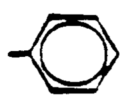 , 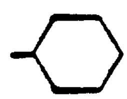 , 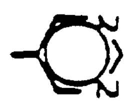 , 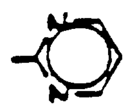 ,

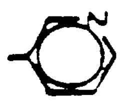 , 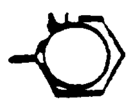 , 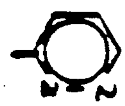

$-M^1$ et $-M^2$

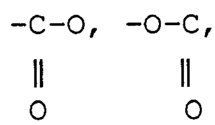

$-CH_2CH_2$, $-CH=CH$, $-CH_2O$, $-OCH_2$,
X représente O ou S.

**3.** Utilisation selon la revendication 2, caractérisée en ce qu'on utilise des esters de l'acide 1,3-dioxolanne-4-carboxylique de formule générale (VI) :

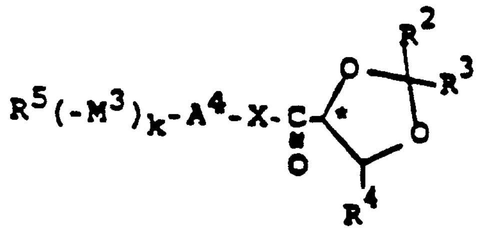 (VI)

dans laquelle :

$R^4$ signifie l'éthyle,

$R^5$ signifie un radical alkyle ou alcényle linéaire ou ramifié comportant de 6 à 12 atomes de carbone, qui peut contenir un atome de carbone asymétrique,

$-M^3$ signifie -O, -S-,

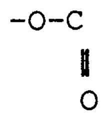 ou 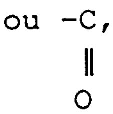,

$-A^4$ signifie

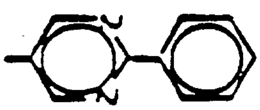, 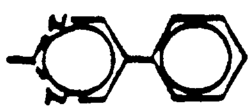, 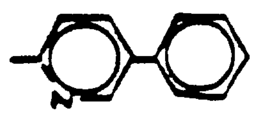,

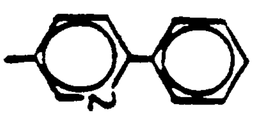, 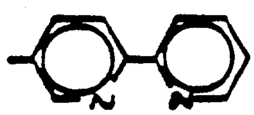, 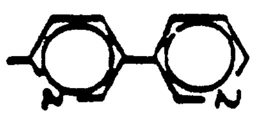,

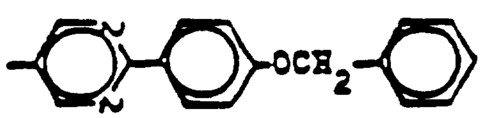, 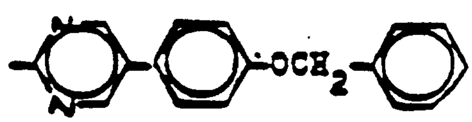,

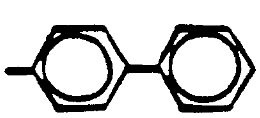, 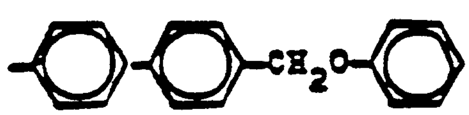,

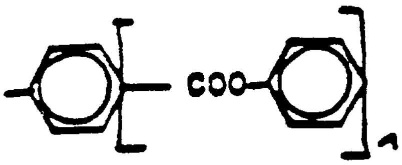 ou 2

**4.** Utilisation selon la revendication 2 ou 3, caractérisée en ce que $R^2$ et $R^3$ sont chaque fois $-CH_3$.

**5.** Esters de l'acide 1,3-dioxolanne-4-carboxylique optiquement actifs de formule générale (V).

**6.** Esters de l'acide 1,3-dioxolanne-4-carboxylique optiquement actifs de formule générale (VI).

**7.** Mélange de cristaux liquides, caractérisé en ce qu'il contient au moins un ester de l'acide 1,3-dioxolanne-5-éthyl-4-carboxylique optiquement actif.

**8.** Mélange de cristaux liquides, caractérisé en ce qu'il contient au moins un ester de l'acide 1,3-dioxolanne-4-carboxylique optiquement actif de formule générale (V).

**9.** Mélange de cristaux liquides, caractérisé en ce qu'il contient au moins un ester de l'acide 1,3-dioxolanne-4-carboxylique optiquement actif de formule générale (VI).

**10.** Elément de commutation ou d'affichage électro-optique contenant un mélange de cristaux liquides selon la revendication 7, 8 ou 9.

**11.** Procédé pour la préparation d'un ester de l'acide 1,3-dioxolanne-4-carboxylique optiquement actif de formule générale (V), caractérisé en ce qu'on fait réagir des phénols ou des thiophénols mésogènes de formule générale (II) :

$R^1(-A^1)_j(-M^1)_k(-A^2)_l(-M^2)_m(-A^3)_n$-X-H

avec des dérivés de l'acide 1,3-dioxolanne-4-carboxylique (III) :

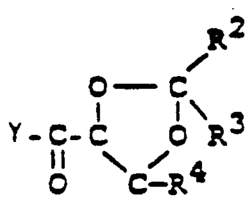

(III)

dans laquelle $R^4$ est un éthyle, en présence d'agents de condensation, Y étant un groupe éliminable.